# EUROPEAN PATENT APPLICATION

(11) **EP 0 586 199 A1**
(43) Date of publication of application: **09.03.1994**
(21) Application number: 93306798.5
(22) Date of filing: 26.08.1993
(51) Int. Cl.: A61M 5/32

(54) **Protective device for injection needle**

(30) Priority: 02.09.1992 US 939057
(71) Applicant: SMITH & NEPHEW INC., Largo, Florida 34643 (US)
(72) Inventor: Scheuble, Gustav A., Des Plaines, Illinois 60018 (US); Kohnke, Fritz, Palatine, Illinois 60067 (US); Paczka, Guenter, Hoffman Estates, Illinois 60195 (US)
(74) Representative: Leckey, David Herbert

(57) **Abstract**

There is described a protective device (10) for an injection needle that can be operatively connected to a syringe, comprising, an elongated sleeve (12), a hub (14) axially moveable in the sleeve (12) between retracted and ready-to-use positions, a hollow needle (18) mounted in the hub (14) and aligned substantially with the longitudinal axis of the sleeve (12), the distal end (20) of the needle (18) being positioned within the sleeve (12) when the hub (14) is in the retracted position and outside the distal end of the sleeve (12) when the needle (18) is in the ready-to-use position; and locking means (26) selectively moveable between the hub (14) and sleeve (12) for locking the hub (14) and sleeve (12) together when the hub (14) is in the retracted position.

## Description

### BACKGROUND OF THE INVENTION

### 1. Field of the Invention

This invention relates to a device that protects health care professionals from accidental contact with used injection needles. More specifically, the invention provides a protective sleeve that permits the health care professional to maintain a cover on an injection needle and only uncover the needle when an injection is administered.

### 2. Description of the Related Art

In recent years, the health care professionals have become increasingly aware of the hazards associated with an accidental contact with an injection needle during resheathing after an injection has been administered. It has been found that numerous diseases, such as AIDS and hepatitis, can be transmitted in this fashion.

Protective sleeves for injection needles have been previously suggested. McFarland, U.S. patent 4,772,272, describes a protective sleeve which covers an injection needle in its retraction or protection position, which allows the needle to be moved so it projects out of the sleeve for administering an injection. The protective sleeve is retained in place in both the retraction and injection positions through a positive frictional fit between a hub which holds the injection needle and the inner surface of the sleeve.

Craig, U.S. patent 2,925,083, describes a hypodermic syringe having a hood for concealing and guarding the injection needle until the syringe is in position for use.

Mitchell, U.S. patent 4,631,057, describes an apparatus for injecting a substance into a human, which has an injection needle coupled to a body. A needle guard is mounted on the body, for movement from a retracted position in which the guard does not shield the needle, to an extended position where the guard shields the needle. The guard can be releasably retained in the retracted position and locked in the extended position. The locking of the needle guard is accomplished by interlocking members carried by the guard and by a collar mounted on the body.

Harbaugh, U.S. patent 4,655,751, describes a liquid dispensing and receiving syringe which includes an elongated cylindrical transparent container open at its front and rear ends and defining a central cavity for holding liquid. A rearwardly extending plunger is slidably secured in the rear end of the cavity for dispensing liquid out the front end of the container and through an injection needle secured to the front end for transporting liquid into the cavity from the needle. A concentric protective shell is connected to and spaced outwardly from the container sidewall and is a slidable between a first needle exposing position and a second needle covering position.

Fox, U.S. patent 4,695,274, describes a safety needle attachment for a syringe body assembly which makes use of a needle holder with an injection needle fixed in the holder. The injection needle is initially surrounded by a protective jacket which is releasably interlocked with the holder. When the needle is used, the interlock is released and the jacket is telescoped over the holder to project the needle through a membrane which covers the end of the jacket.

Haber et al., U.S. patent 4,767,413, describes a disposable dental syringe having a prefilled ampule of liquid in a double-ended injection needle arranged and spaced in axial alignment with the ampule. The ampule is moveable axially through the cylinder of the syringe until the proximal end of the needle penetrates a ampule and the distal end of the needle extends outwardly from the cylinder for administering an injection. The ampule is locked in the distal position so that an injection may be administered. Upon completing the injection, the ampule is released from the distal position and moved proximally through the cylinder, so that the distal end of the needle is automatically retracted within the cylinder.

There is a need for a protective sleeve for an injection needle that is convenient to use, light in weight, and that will minimize the risk of health care professionals coming in contact with used needles by making sure the needle is effectively locked in a retracted position within the sleeve when the needle is not being used.

### SUMMARY OF THE INVENTION

The invention provides a protective device for an injection needle that is universal in the sense that it can be made to fit most syringes, so that it can be used in a wide variety of applications. Further, the protective device is designed to minimize interference with the manipulations required to change a carpule or ampule in a syringe when the protective device is in place.

Since the device is preassembled into a self-contained structure, the health care professional does not have to assemble any parts of the protective device. All that is necessary, is to position the device onto the threaded portion of the distal end of a syringe and screw it into place, in a manner similar to screwing a conventional injection needle into place.

The protective device includes an elongated sleeve which has open distal and proximal ends. The proximal end is adapted to cooperate with the distal end of a syringe. A hub assembly is slidably contained within the protective sleeve. A hollow needle for administering injections is axially connected to the hub such that a rear portion of the needle projects from the proximal side of the hub for penetrating a carpule which contains anesthetic or a medicament. The major or front portion of the needle, bearing an injection tip, projects from the distal side of the hub.

The hub has a threaded opening which can be screwed onto the threaded distal end of a syringe. The inner surface of the protective sleeve has a guide for preventing rotation of the hub within the sleeve and for guiding the sliding of the hub within the sleeve. Further, the sleeve and hub have a cooperating releasable lock mechanism in the form of a flexible, outwardly-urged tab for locking the hub in a first, needle-retracted position within the sleeve such that the tip of the injection needle does not project beyond the distal end of the sleeve.

In its preferred embodiment, the invention provides a one-piece hub, preferably fabricated from an organic polymeric material, to which the injection needle is fixedly, axially attached with an adhesive. The hub has a pair of tabs formed integral with the hub which project into openings formed in the sleeve for locking the hub in the needle-retracted position. The tabs have enough flexibility so they can easily be pushed inwardly by the user to release them from the openings in the sleeve so the hub and needle can be moved to the ready-to-use position.

The sleeve is sized to contain the full length of the frontal tip-bearing portion of the needle when it is in the retracted position. To expose the needle tip, the locking mechanism is released and the sleeve is pulled toward the syringe. The freed hub is guided toward the distal end of the sleeve so that the tip of the needle projects beyond the opening in the distal end of the sleeve. After the patient has been injected, the user is protected from the needle because the sleeve is moved forward over the needle, until the cooperating locking means on the hub and sleeve engage to again lock the needle in the retracted position.

### BRIEF DESCRIPTION OF THE DRAWINGS

FIGURE 1 is a cross-sectional view of an embodiment of the invention protective sleeve with an injection needle in a retracted position.

FIGURE 2 is a cross-sectional view along section line 2-2 of Fig. 1.

FIGURE 3 is a cross-sectional view along section line 3-3 of Fig. 1.

FIGURE 4 is a cross-sectional view of an embodiment of the invention protective sleeve with the injection needle in the ready-to-use position.

FIGURE 5 is a plan view, partially in section, of the subject protective sleeve in position for attaching to a syringe, with a carpule of anesthetic ready to be inserted into the syringe.

FIGURE 6 is a plan view, partially in section, of the protective sleeve syringe assembly with the sleeve drawn and the injection needle in the ready-to-use position.

FIGURE 7 is a plan view, partially in section, of a protective sleeve syringe assembly with the injection needle in its needle-retracted position.

### DETAILED DESCRIPTION OF AN EXEMPLARY EMBODIMENT

The present invention is directed to a protective device for an injection needle for protecting health care professionals from contact with the needle when the needle is not being used.

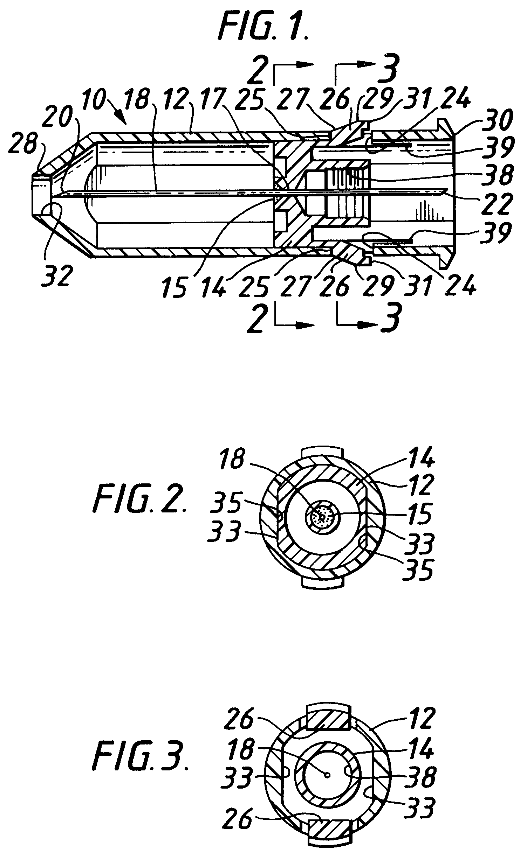

Figs. 1-7 show a preferred embodiment of the protective device of the present invention. The protective device 10 is shown in detail in Figs. 1-4, and includes a generally cylindrical sleeve 12, which is constructed of a transparent polymeric material and has openings at its proximal and distal ends 30, 32.

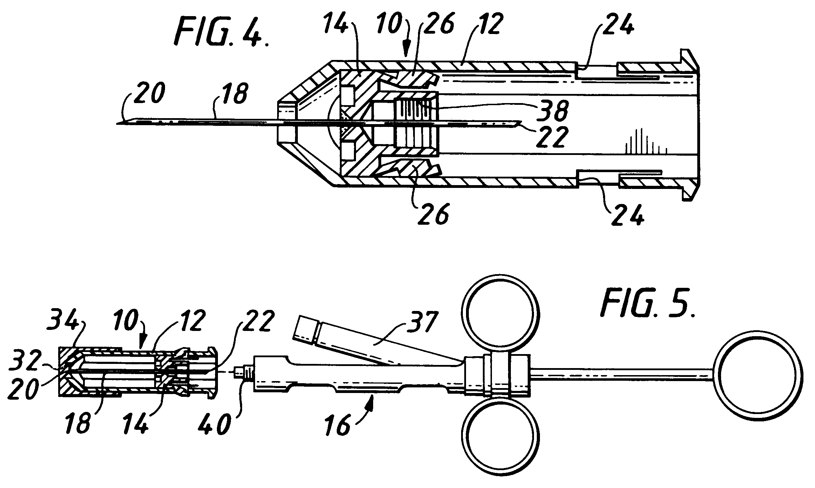

A sliding hub 14, which is adapted to be connected to a syringe 16 (see Figs. 5-7), is slidably moveable in the sleeve 12. An injection needle 18 has a distal end 20 which is used for injections, and a proximal end 22 for penetrating into a carpule filled with anesthetic or a medicament such as the carpule 37 shown in Fig. 5. The needle 18 is securely mounted in the hub 14, preferably through the application of an adhesive 15 after the needle 18 has been inserted through an opening 17 formed axially through the hub 14. Adhesives found to be suitable include known epoxy resins.

The hub 14 is formed of a unitary piece of polymeric material, with the needle 18 connected directly to the hub 14 as described. The hub 14 also has a threaded opening 38 which engages a threaded end 40 of a syringe 16 for connecting the hub 14 to the syringe 16 as shown in Figs. 5-7.

A locking mechanism is provided for locking the hub 14 to the sleeve when the needle 18 is in the retracted position of Fig. 1 for preventing the needle 18 from being exposed when it is not being used. When the hub 14 and the needle 18 are in their retracted position (Fig. 1), the sleeve 12 extends over and covers both the distal end 20 and the proximal end 22 of the needle 18 to protect the health care professional who will administer an injection from any contact, accidental or otherwise, with the injection needle 18.

In the preferred embodiment of the locking mechanism, a pair of generally rectangular openings 24 are formed in the wall of the sleeve 12. A pair of tabs 26 are formed as part of the hub assembly 14 and are designed to extend into and cooperate with the openings 24. The tabs 26 operate to retain the needle 18 in the retracted position until it is released as described below.

The tabs 26 are formed as integral part of the hub 14. Because of their shape and the polymeric or other suitable material used to form the hub 14, the tabs 26 are flexible enough so they can easily be moved inwardly to release the hub 14 from its positive engagement with the sleeve by simply pushing on them. The tabs 26 each have an upper ledge 27 which engages an edge 25 of each opening 24 for locking the hub 14 in place. The outer side 29 of the tab 26 is formed at a slant with the lower end 31 projecting beyond the outer surface of the sleeve 12 as shown in Fig. 1.

This design permits a slight amount of inward pressure exerted on the tabs 26 by the user to cause the ledge 27 to disengage from the edge 25. A slot 39 extends from each proximal corner of the openings 24 to add flexibility in that portion of the sleeve 12 to make sure that the user can easily disengage the tabs 26 from the openings 24 when they are pushed inward.

This pushing action unlocks the hub 14 and allows it to be moved as described below. The positive engagement is effective in preventing the hub 14 from inadvertently being pushed outward to expose the needle 18.

When the tabs 26 are pushed inwardly, the hub 14 is disengaged from the sleeve 12. This allows the hub assembly to slide within the sleeve 12 toward the opening in the distal end 32. The inner surface of the sleeve 12 is formed with flat sections 33 (see Fig. 2) which cooperate with flat sides 35 on the hub 14 so that the hub 14 does not move relative to the sleeve 12 in a non-axial direction as the hub 14 moves toward the distal end 32. In this way, the distal end 20 of the needle 18 is moved through the opening 32 to facilitate injection in a patient. Afterwards, the needle 18 is retracted and locked in place within the sleeve 12 as described below.

As illustrated in Figs. 1 and 5, the distal end 28 of the sleeve 12 has a central opening 32, which can be adapted to receive a top sealing means or cap 34 which covers the distal end 28 and fits into the opening 32. The cap 34 is preferably tamper indicative to maximize safety of the device to the user and patient. The sleeve 12 also has a proximal end 30 which is adapted to receive a bottom sealing means or cap (not shown) which can be a flat, circular piece having the same outside diameter as the sleeve 12. A circular ridge which has an outside diameter the same as the bottom inside diameter of the protective sleeve 12 is formed perpendicular to the flat portion. The top sealing means 34 and the bottom sealing means may preferably be interconnected by a strap or other suitable connecting means.

Figs. 5-7 illustrate how the protective device 10 is connected to a syringe 16. The syringe 16 is fabricated of known materials and has a cavity 36 adapted to receive a carpule 37 containing the anesthetic or other medicament to be administered to a patient by injection.

In operation, as shown in Fig. 5, the syringe 16, with the carpule 37 loaded in its cavity 36, is inserted into the bottom end 30 of the protective sleeve 12 until it stops. The protective sleeve 12 is then rotated clockwise until the threaded opening 38 is threaded onto the threaded end 40 of the syringe 16. In this position, the proximal end 22 of the needle 18 has penetrated the carpule 37 and the syringe 16 is ready for use.

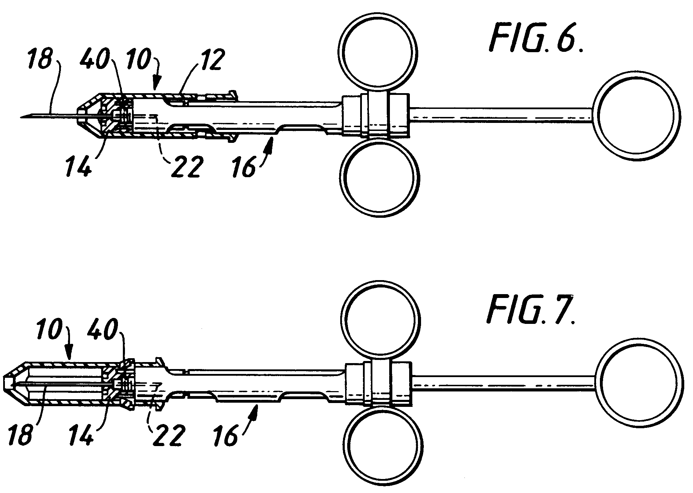

When an injection is to be made, the top cap 34 is removed. The health professional holds the sleeve 12 between his or her thumb and forefinger at the point where the tabs 26 extend through the openings 24, which is about two-thirds of the length of the sleeve 12, from distal end 28 to proximal end 30. The tabs 26 are then manually depressed, releasing the hub assembly 14. The syringe 16 is pushed through the sleeve 12 to extend the needle 18 through the opening 32 to the ready-to-use position shown in Fig. 6, and the injection is made.

When the injection is complete, the protective sleeve 12 is held firmly by the health care professional between the thumb and forefinger of one hand while the other hand holds the syringe 16. The syringe 16 is pulled back to the position shown in Fig. 7 to retract the needle 18 until the tabs 26 are in their original position where they extend through the openings 24 to lock the sleeve 12 in a retracted position. The cooperating flat sections 33, 35 on the sleeve 12 and the hub 14 prevent any relative non-axial movement between them so that the tabs 26 engage the openings 24 to lock the needle 18 in its retracted position.

While still holding the protective sleeve 12, the health care professional rotates the protective sleeve counterclockwise until the threaded end 40 of the syringe 16 disengages from the threaded portion 38 of the hub assembly 14. The sleeve 12 and the hub 14 with the needle 18 are then disposed of by means well known in the art.

As shown best in Fig. 1, after the protective sleeve has been removed from the syringe 16, both the distal and proximal ends 20, 22, of the needle 18 are totally contained within the sleeve 12 in order to minimize accidental contact with the injection needle 18. The locking action provided between the tabs 26 and the openings 24 minimizes any inadvertent exposure of the injection needle 18. The protective device of the present invention when used properly as described above virtually eliminates the possibility of the health care professional sticking himself or herself during resheathing of the needle.

The foregoing is illustrative of the present invention but not limiting. Numerous variations and modifications may be effective without departing from the true scope and spirit of the invention.

## Claims

1. A protective device for an injection needle that can be operatively connected to a syringe, comprising:
(a) an elongated sleeve with a longitudinal axis between open proximal and distal ends;
(b) a hub axially moveable in the sleeve between retracted and ready-to-use positions, the hub being adapted for connection with said syringe;
(c) a hollow needle mounted in the hub and aligned substantially with the longitudinal axis of the sleeve, the needle having a distal end projecting from one end of the hub and a proximal end projecting from the other end of the hub;
(d) the distal end of the needle being positioned within the sleeve when the hub is in the retracted position and outside the distal end of the sleeve when the needle is in the ready-to-use position; and
(e) locking means selectively moveable between the hub and sleeve for locking the hub and sleeve together when the hub is in the retracted position.

2. The device of claim 1, wherein the sleeve comprises a generally cylindrical body formed of a transparent polymeric material.

3. The device of claim 1, wherein the sleeve includes a tapered distal end.

4. The device of claim 1, wherein the hub is a unitary structure formed of a polymeric material.

5. The device of claim 4, wherein the needle extends through an opening in the hub and is secured therein by a hardened adhesive.

6. The device of claim 1, wherein the hub includes a threaded opening adapted to engage a cooperating threaded end on a syringe.

7. The device of claim 1, wherein the locking means includes at least one flexible, outwardly urged tab on the hub and an opening in the sleeve for receiving the tab.

8. The device of claim 7, wherein the hub includes a pair of tabs on opposing sides.

9. The device of claim 1, wherein the sleeve and hub include cooperating guide means for preventing relative non-axial movement as the hub moves between the retracted and ready-to-use positions.

10. The device of claim 1, wherein the guide means includes at least one flat portion formed on the inner surface of the sleeve and a cooperating flat portion formed on the hub.

11. An improved hub for holding an injection needle of the type wherein the hub is slidably moveable between retracted and ready-to-use positions in an elongated protective sleeve, the needle being adapted for connection to a syringe for delivery of anesthetic or a medicament, the improvement comprising:
(a) a hub formed of a unitary construction and adapted for axial movement in the protective sleeve;
(b) an opening in the hub parallel to the direction of movement of the hub;
(c) a hollow needle extending through the opening with a distal end projecting from one end and a proximal end projecting from the other end of the hub; and
(d) means for securing the needle in the opening.

12. The hub of clam 11, wherein the hub is formed of a polymeric material.

13. The hub of claim 11, and further including a pair of flexible tabs projecting from opposite sides for locking into openings formed in the sleeve.

14. The hub of claim 11, wherein the means for securing includes a hardened adhesive.

15. The hub of claim 1, and further including at least one flat surface parallel to the direction of hub movement for cooperating with a flat surface on the sleeve to prevent relative non-axial movement.
